# DEMANDE DE BREVET EUROPEEN

(11) **EP 1 514 524 A1**
(43) Date de publication de la demande: **16.03.2005**
(21) Numéro de dépôt: 04292168.4
(22) Date de dépôt: 09.09.2004
(51) Int. Cl.: A61B 19/08

(54) **Champ opératoire conditionné et procédé pour le préparer**

(30) Priorité: 10.09.2003 FR 0310643
(71) Demandeur: VYGON, F-95440 Ecouen (FR)
(72) Inventeur: Algrain, Isabelle, 95380 Puiseux en France (FR); Hocq, Xavier, 59530 Villers Pol (FR); Lestoquoy, Patrick, 59551 Attiches (FR)
(74) Mandataire: Schrimpf, Robert

(57) **Abrégé**

Le champ opératoire est conditionné dans une feuille de réemballage permettant la stérilisation, cette feuille étant pliée de façon à déterminer un panneau de dessous (2a) sur lequel est déposé le champ (1), et un petit (2b) et un grand (2c) panneaux de dessus qui coopèrent pour recouvrir le champ, ledit petit panneau (2b) étant replié sur lui-même vers l'extérieur pour constituer un volet (2d) de préhension, et ledit grand panneau (2c) étant appliqué sur ledit volet pour le recouvrir partiellement et étant lui-même replié vers l'extérieur pour constituer un volet de préhension (2e), les panneaux de dessus étant fixés au panneau de dessous entre le champ opératoire et les bords transversaux du panneau de dessous, et ledit grand volet étant fixé au volet du petit panneau dans la zone du volet qu'il recouvre.

## Description

L'invention concerne le conditionnement des champs opératoires et un procédé d'emballage d'un champ dans une feuille dite de réemballage.

Une technique connue de conditionnement consiste à mettre le champ dans une feuille de qui est pliée autour du champ, l'ensemble étant placé dans un blister souple pour être envoyé en stérilisation.

Ce pliage est long et coûteux et obéit à des règles de pliage strictes.

Arrivé à l'entrée du bloc opératoire, le champ conditionné est sorti du blister par une infirmière non stérile qui déplie ensuite la feuille de réemballage sans toucher l'intérieur de celle-ci ni le champ opératoire et qui la présente à une infirmière stérile qui peut prendre sans risque le champ opératoire sans qu'il ait été contaminé.

La publication US 4 342 392 divulgue un champ opératoire conditionné dans une feuille pliée de façon à déterminer un panneau de dessous (38) présentant deux bords longitudinaux et deux bords transversaux et sur lequel est déposé le champ, et deux panneaux de dessus (58,54) attenant respectivement à l'un et à l'autre des bords longitudinaux du panneau de dessous et qui coopèrent pour recouvrir le champ, l'un des panneaux de dessus (58) étant replié sur lui-même vers l'extérieur pour constituer un volet (36) et l'autre panneau de dessus étant appliqué sur ce volet.

Selon cette publication, l'autre panneau de dessus (55) est replié une première fois sur lui-même pour doubler le panneau et replié une deuxième fois mais sous le panneau de dessous (38) pour constituer un volet de préhension (32).

La fermeture du conditionnement est assurée par le pliage de panneaux latéraux (68) sur les panneaux de dessus et sécurisation éventuelle de la fermeture par des rubans adhésifs (76) ou des points de colle.

La consommation de matière, l'encombrement et le poids d'un tel conditionnement constituent des handicaps.

En outre, la manipulation du conditionnement pour son pliage et son dépliage est relativement compliquée et implique des déplacements de main et des mouvements qui doivent être bien contrôlés pour éviter de toucher le champ emballé.

La présente invention contribue à minimiser ces handicaps et à faciliter le pliage et l'ouverture du conditionnement et l'accès au champ.

Selon l'invention, le champ opératoire est conditionné dans une feuille de réemballage permettant la stérilisation, cette feuille étant pliée de façon à déterminer un panneau de dessous présentant deux bords longitudinaux et deux bords transversaux et sur lequel est déposé le champ, et deux panneaux de dessus attenant respectivement à l'un et à l'autre des bords longitudinaux du panneau de dessous et qui coopèrent pour recouvrir le champ, l'un des panneaux de dessus ou petit panneau étant replié sur lui-même vers l'extérieur pour constituer un volet de préhension intégralement situé sur le dessus du champ, et l'autre des panneaux de dessus ou grand panneau étant appliqué sur ledit volet, caractérisé en ce que le grand panneau ne recouvre que partiellement le volet du petit panneau et lui est fixé dans la zone du volet qu'il recouvre, en ce que le grand panneau est lui-même replié vers l'extérieur pour constituer un volet de préhension intégralement situé sur le dessus du champ, et en ce que les panneaux de dessus sont fixés au panneau de dessous dans des zones du panneau de dessous comprises entre le champ et les bords transversaux du panneau de dessous.

Dans des réalisations préférées, les fixations sont réalisées par scellage à chaud ou à froid. Dans le cas d'un scellage à froid par simple pression, la feuille est enduite aux endroits voulus d'un adhésif permettant le scellage ; dans le cas d'un scellage à chaud, la feuille est enduite d'une laque ou d'une colle réagissant dans les zones de pression avec apport de chaleur.

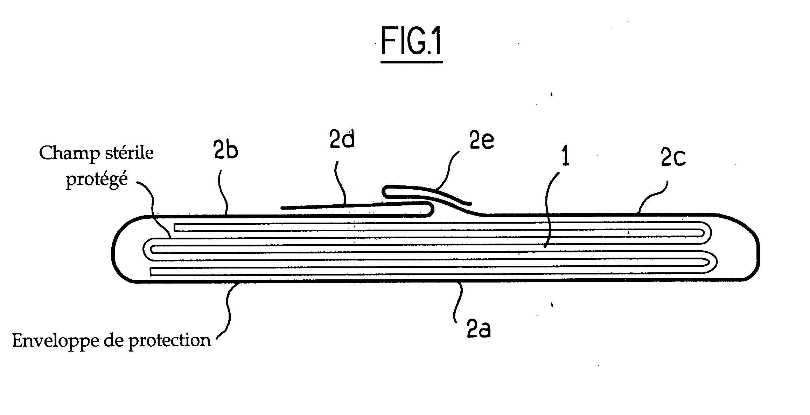

On décrira ci-après un mode de réalisation d'un conditionnement conforme à l'invention en référence aux figures du dessin joint sur lequel
La figure 1 est une vue en coupe transversale d'un champ emballé selon l'invention ;

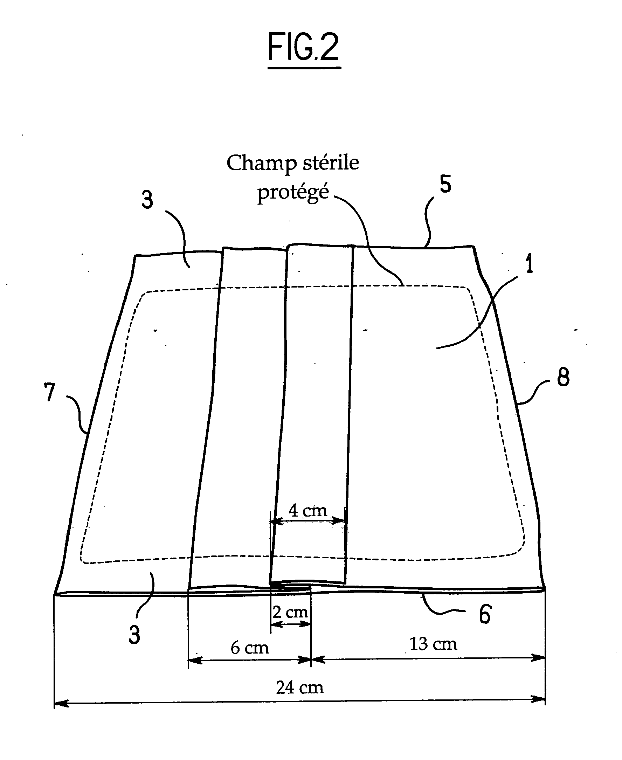

la figure 2 est une vue de dessus en perspective du champ emballé ;
les figures 3 et 4 sont deux vues successives d'une opération d'ouverture de l'emballage, et
les figures 5 et 6 sont deux vues successives d'une variante d'opération d'ouverture de l'emballage.

On a représenté sur les figures un champ opératoire (1) emballé dans une feuille de papier (2) permettant la stérilisation, par exemple un papier poreux laissant passer et sortir un gaz tel que l'oxyde d'éthylène , scellable à chaud ou à froid. On n'a pas représenté l'enveloppe externe ou blister dans laquelle est placé le champ conditionné dans sa feuille.

La feuille est pliée de façon à déterminer un panneau de base (2a) sur laquelle repose le champ et deux panneaux de dessus (2b) et (2c) qui se font face et qui recouvrent le champ.Le panneau de dessus (2b) ou « petit panneau » est appliqué sur le panneau de base et replié sur lui-même vers l'extérieur pour constituer un volet de préhension (2d) ; le panneau de dessus (2c) est appliqué sur le panneau de base et en partie sur le volet du petit panneau puis est rabattu vers l'extérieur pour constituer un volet de préhension (2e).

Les deux volets sont situées sur le dessus du champ aptes à être saisis pour écarter l'un et/ou l'autre des panneaux de dessus et accéder au champ opératoire.

Les panneaux de dessus sont soudés au panneau de base dans les zones de bordure (3) entre le champ stérile et les bords transversaux (4,5) du panneau de base ; en outre le grand panneau (2b) est soudé au volet(2d) du petit panneau.

Dans une réalisation préférée
- la longueur des bords transversaux (4,5) du panneau de base est de 24 cm ;
- la longueur correspondante du petit panneau de dessus est de 11 cm ;
- la longueur correspondante du grand panneau de dessus est de 13 cm ;
- la longueur correspondante du volet du petit panneau est de 6 cm ;
- la longueur correspondante de chevauchement de ce volet par le grand panneau est de 2 cm ;
- la longueur correspondante du volet du grand panneau est de 4 cm ;
- la largeur de scellement est de 2 cm.

Il va de soi que ces dimensions sont données à titre approximatif et non limitatif.

Il va de soi également que la feuille est généralement d'un format rectangulaire.

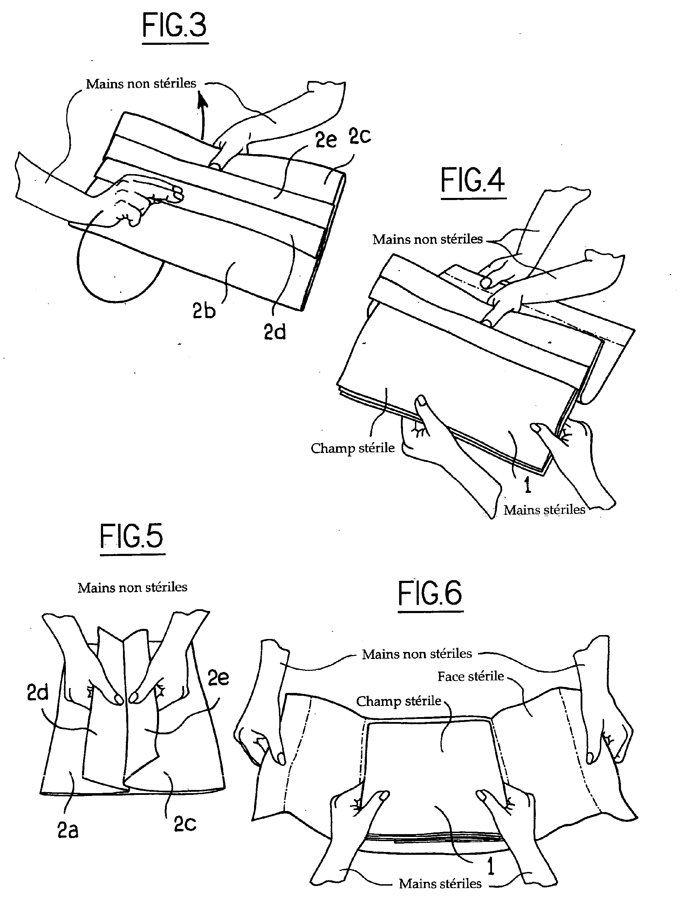

Sur les figures 3 et 4, l'infirmière non stérile a sorti le champ emballé du blister , tire sur le volet (2e) du grand panneau de dessus et replie la feuille en dessous du champ sans le toucher. L'infirmière stérile peut alors très facilement saisir le champ et le tirer vers elle, l'infirmière non stérile relâchant un peu la pression manuelle sur l'ensemble feuille/champ et conservant la feuille vide à la fin.

Dans l'autre manière illustrée sur les figures 5 et 6, l'infirmière non stérile tire sur les volets en même temps et déplie complètement la feuille de réemballage, présentant le champ sur le panneau de base de cette feuille à l'infirmière stérile : ni la face intérieure de l'emballage,ni le champ n'ont été touchés par l'infirmière non stérile.

L'invention n'est pas limitée au mode de réalisation qui a été décrit. Par exemple, la feuille de réemballage peut comporter des lignes d'affaiblissement destinées à faciliter les pliages.

L'invention concerne également un procédé pour l'emballage d'un champ opératoire dans une feuille de façon à obtenir un emballage comme décrit ci-dessus.

Selon l'invention, on met en défilement une nappe de papier ayant une largeur correspondant à la longueur transversale de la feuille à plier, on rabat vers le bas les bords longitudinaux de la nappe pour former les dits volets, on dépose un champ stérile sur la nappe, on plie la nappe entre le champ et l'un des volets pour former le petit panneau de dessus que l'on applique sur une partie du champ, on plie ensuite la nappe entre le champ et l'autre volet pour former le grand panneau de dessus que l'on applique sur le reste du champ et sur le volet du petit panneau de dessus, et on réalise les scellements.

Les pliages sont réalisés aux endroits appropriés compte tenu des dimensions voulues pour les panneaux et les volets, et le recouvrement désiré du volet du petit panneau de dessus par le grand panneau de dessus. Pour faciliter les pliages, notamment ceux destinés à former les panneaux de dessus, il est avantageux de réaliser des amorces de pliage aux endroits voulus dans la nappe, par exemple au moyen de molettes qui réalisent en continu des affaiblissements de la paroi de la nappe au cours du défilement de la nappe.

Le défilement de la nappe peut être continu ou discontinu et comporte des étapes de découpe transversale pour séparer les emballages successifs . les champs ainsi conditionnés sont mis sous blister et stérilisés. L'invention n'est pas limité à ce procédé d'emballage qui n'est décrit qu'à titre d'exemple préféré.

## Revendications

1. Champ opératoire conditionné dans une feuille de réemballage permettant la stérilisation, cette feuille étant pliée de façon à déterminer un panneau de dessous (2a) présentant deux bords longitudinaux (6,7) et deux bords transversaux (4,5) et sur lequel est déposé le champ (1), et deux panneaux de dessus (2b,2c) attenant respectivement à l'un et à l'autre des bords longitudinaux du panneau de dessous et qui coopèrent pour recouvrir le champ, l'un des panneaux de dessus ou petit panneau (2b) étant replié sur lui-même vers l'extérieur pour constituer un volet de préhension (2d) intégralement situé sur le dessus du champ, et l'autre des panneaux de dessus ou grand panneau (2c) étant appliqué sur ledit volet, **caractérisé en ce que** le grand panneau ne recouvre que partiellement le volet du petit panneau et lui est fixé dans la zone du volet qu'il recouvre, **en ce que** le grand panneau est lui-même replié vers l'extérieur pour constituer un volet de préhension (2e) intégralement situé sur le dessus du champ, et **en ce que** les panneaux de dessus sont fixés au panneau de dessous dans des zones (3) du panneau de dessous comprises entre le champ et les bords transversaux du panneau de dessous.

2. Champ opératoire selon la revendication 1 dans lequel les fixations sont réalisées par scellage.

3. Champ opératoire selon la revendication 1 ou 2 dans lequel la feuille de réemballage présente des zones affaiblies pour faciliter les pliages.

4. Champ opératoire selon l'une des revendications 1 à 3 dont la feuille de réemballage est en papier.

5. Champ opératoire selon l'une des revendications 1 à 4 contenu dans un blister.

6. Procédé pour l'emballage d'un champ opératoire de façon à obtenir un champ opératoire conditionné conformément à l'une des revendications 1 à 5, dans lequel on met en défilement une nappe de papier ayant une largeur correspondant à la longueur transversale de la feuille avant pliage, on rabat vers le bas les bords longitudinaux de la nappe pour former les dits volets, on dépose un champ stérile sur la nappe, on plie la nappe entre le champ et l'un des volets pour former le petit panneau de dessus que l'on rabat sur une partie du champ, on plie ensuite la nappe entre le champ et l'autre volet pour former le grand panneau de dessus que l'on applique sur le reste du champ et sur le volet du petit panneau de dessus, et on réalise les scellements.
